(19) Europäisches Patentamt / European Patent Office / Office européen des brevets

(11) **EP 2 295 136 A1**

(12) **EUROPEAN PATENT APPLICATION**
published in accordance with Art. 153(4) EPC

(43) Date of publication:
**16.03.2011 Bulletin 2011/11**

(51) Int Cl.:
***B01J 8/02*** (2006.01)  ***C07C 51/25*** (2006.01)

(21) Application number: **09773310.9**

(22) Date of filing: **18.06.2009**

(86) International application number:
**PCT/JP2009/061096**

(87) International publication number:
**WO 2010/001732 (07.01.2010 Gazette 2010/01)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **30.06.2008 JP 2008170180**

(71) Applicant: **Nippon Shokubai Co., Ltd.**
**Osaka-shi, Osaka 541-0043 (JP)**

(72) Inventors:
• **TANIMOTO, Michio**
**Himeji-shi**
**Hyogo 671-1214 (JP)**
• **HAKOZAKI, Nobuyuki**
**Himeji-shi**
**Hyogo 671-1143 (JP)**
• **MIGITA, Yusaku**
**Himeji-shi**
**Hyogo 671-1242 (JP)**

(74) Representative: **Müller-Boré & Partner**
**Patentanwälte**
**Grafinger Straße 2**
**81671 München (DE)**

(54) **METHOD OF PACKING SOLID PARTICULATE SUBSTANCE INTO FIXED-BED MULTITUBULAR REACTOR**

(57)    A method of loading solid particles into reaction tubes of a fixed-bed multitubular reactor, comprising the step of loading two or more kinds of solid particles into each of the reaction tubes so that the respective kinds of the solid particles are stacked in layers in a direction of an axis of the tube, wherein a gaseous fluid is introduced into the reaction tubes at least once every time the each kind of the solid particles is loaded in the layer.

Fig. 1

Catalyst (1)

Catalyst (2)

Ceramic balls (1)

Reaction gas

**Description**

TECHNICAL FIELD

The present invention relates to a method of loading solid particles such as a catalyst into a fixed-bed multitubular reactor.

BACKGROUND ART

As a method of loading solid particles such as a catalyst into a fixed-bed multitubular reactor, there is generally employed a method of dropping solid particles from an upper end opening of each reaction tube to load the particles into a reactor. In this method, however, a part of the solid particles may be disrupted or powdered due to physical impact upon dropping, and powdered particles resulting therefrom may increase a pressure loss or cause a substantive reduction of the amount of a catalyst to be engaged in a reaction. In addition, the powdered particles may cause a reduction of a yield of a target product or a catalyst lifetime. Moreover, the powdered particles may cause blockage of the reaction tubes due to by-products resulting from a nonuniform reaction in the reactor.
As a way to solve these problems, various loading methods have been proposed, as well as a method of enhancing the mechanical strength of the solid particles themselves such as a catalyst. For example, Patent Literature 1 discloses a method of dropping a solid catalyst from an upper end of a reaction tube while discharging a gas from a lower end of a tubular article which is inserted from an opening of the upper end of the reaction tube; and then, pulling up the inserted tubular article, thereby loading the solid catalyst. Patent Literature 2 discloses a method of loading a catalyst into a reactor by dropping while placing a string-like article having a shape and a thickness which do not inhibit dropping of the catalyst in the reactor. Patent Literature 3 discloses a method of dropping a pellet-like catalyst from a top of a reaction tube to load into a multitubular reactor while flowing air upward from a bottom of the reaction tube at a velocity about 1/2 to twice the minimum fluidizing velocity. Patent literature 4 discloses a method of maintaining a moisture content of an atmosphere gas in a reaction tube in a range of 0.3 mass % to 1.2 mass % upon loading a solid catalyst into a reactor by dropping.

CITATION LIST

PATENT LITERATURE

PATENT LITERATURE 1
Japanese Unexamined Patent Application Publication No. 2003-340266
PATENT LITERATURE 2
Japanese Unexamined Patent Application Publication No. 6-7669
PATENT LITERATURE 3
Japanese Unexamined Patent Application Publication No. 62-30545
PATENT LITERATURE 4
Japanese Unexamined Patent Application Publication No. 2005-224661

SUMMARY OF INVENTION

TECHNICAL PROBLEM

According to the methods disclosed in the above Patent Literatures 1 to 4, a catalyst is suppressed to be disrupted or powdered when it is loaded into a reactor; however, it is impossible that the disruption or powdering is completely suppressed, and therefore, not a little amount of the powdered particles generated upon loading the catalyst should present in reaction tubes after the loading operation. As a result, a pressure loss of the respective reaction tubes increases and a substance variability in the amount of the catalyst filled in the reaction tubes among the tubes is caused, whereby a reduction in a yield of a target product or a reduction in a catalyst lifetime may be caused due to nonuniformity of reaction conditions, or blockage of the reaction tubes may be caused due to by-products. Further, even if the pressure loss of the reaction tubes is adjusted to be within a certain range immediately after the loading of solid particles such as a catalyst, there is a big problem that the variability of the pressure loss among the reaction tubes may be caused after a reaction is continued for a long period.
Therefore, an object of the present invention is to provide a method of effectively removing powdered particles, which is generated in not a little amount upon loading solid particles such as a catalyst into the fixed-bed multitubular reactor, from reaction tubes by a simple and easy method, thereby stably operating a fixed-bed multitubular reactor for a long period.

SOLUTION TO PROBLEM

The method of loading solid particles of the present invention which solves the above problems is a method of loading solid particles into reaction tubes of a fixed-bed multitubular reactor, comprising the step of loading two or more kinds of solid particles into each of the reaction tubes so that the respective kinds of the solid particles are stacked in layers in a direction of an axis of the tube, wherein a gaseous fluid is introduced into the reaction tubes at least once every time the each kind of the solid particles is loaded in the layer. According to the method of the present invention, powdered particles generated upon loading the solid particles can be removed effectively, a pressure loss can be easily adjusted, and further, increase of the pressure loss and variability of the pressure loss among the reaction tubes with time can be suppressed. As a result, when a gas-phase catalytic oxidation reaction is conducted by using such fixed-bed multitubular reactor, it is possible to stably obtain a target product at a high yield for a long period.

ADVANTAGEOUS EFFECTS OF INVENTION

According to the present invention, powdered particles generated upon loading the solid particles can be easily removed from the reaction tubes; and as a result, a pressure loss of the fixed-bed multitubular reactor becomes low, the pressure loss can be easily adjusted, increase of the pressure loss and variability of the pressure loss among the reaction tubes with time can be suppressed, and a target product can be stably obtained at a high yield for a long period by using the fixed-bed multitubular reactor.

BRIEF DESCRIPTION OF DRAWINGS

Fig. 1 shows a schematic view of a manner of loading solid particles in Example 1.
Fig. 2 shows a schematic view of a manner of loading solid particles in Example 10.

DESCRIPTION OF EMBODIMENTS

The present invention will hereinafter be described in detail; however, the present invention is not limited to the following description, and can be put into practice after appropriate modification or variations within a range meeting the gist of the present invention in addition to the following embodiments.
According to a method of loading solid particles of the present invention, in loading solid particles into reaction tubes of a fixed-bed multitubular reactor, two or more kinds of solid particles are loaded into each of the reaction tubes so that the respective kinds of the solid particles are stacked in layers in a direction of an axis of the tube, wherein a gaseous fluid is introduced into the reaction tubes at least once every time the each kind of the solid particles is loaded in the layer. Thus, the method of loading solid particulars of the present invention comprises the steps of: loading solid particles into each of reaction tubes of a fixed-bed multitubular reactor so that the solid particles are stacked in a layer in a direction of an axis of the tube, as a loading step; and introducing a gaseous fluid into the reaction tubes after the loading step, as a gas introducing step; wherein the loading step and the gas introducing step are respectively conducted twice or more times.
When a gaseous fluid is introduced into the reaction tubes after the whole amount of the solid particles required for each of the reaction tubes is loaded thereinto as in the conventionally known methods, it is difficult to completely remove powdered particles generated upon loading, since the pressure loss has already become high. Meanwhile, according to the present invention, the powdered particles generated upon loading can be removed efficiently by introducing a gaseous fluid into the reaction tubes every time each layer of the solid particles is loaded into the reaction tubes. Further, since the powdered particles generated upon loading the solid particles can be removed effectively, the pressure loss of the reaction tubes of the fixed-bed multitubular reactor can be easily adjusted, and increase of the pressure loss and variability of the pressure loss among the reaction tubes with time can be suppressed. As a result, when a gas-phase catalytic oxidation reaction is conducted by using such fixed-bed multitubular reactor, it is possible to stably obtain a target product at a high yield for a long period.
The fixed-bed multitubular reactor usable in the present invention is not particularly restricted; however, a general reactor having reaction tubes with a circular cross-sectional shape is used. An inner diameter (D) of the reaction tube is not particularly limited; however, it is preferably in a range of 15 mm to 50 mm, more preferably 20 mm to 40 mm, and even more preferably 22 mm to 38 mm. A length of the reaction tube is determined depending on a capability of associated apparatuses and the like, and can be appropriately selected from a range of 1 m to 10 m.

Examples of the solid particles used in the present invention include, for example, a catalyst, an inert substance and the like. Preferably, a catalyst is used as at least one kind of the solid particles.

When a catalyst is used as the solid particles, the kind of the catalyst is not particularly restricted as long as it can be used by being loaded into a fixed-bed multitubular reactor. For example, well-known catalysts described in the following items (1) to (9) can be used.

(1) A catalyst for producing ethylene oxide by oxidizing ethylene in a gas phase, that contains silver as an essential element (see Japanese Unexamined Patent Application Publication Nos. 63-116743, 62-4444, 5-329368, 10-510212, 5-84440, and the like).

(2) A catalyst for producing (meth)acrolein or (meth)acrylic acid by oxidizing propylene, isobutylene, tertiary butanol, and/or methyl tertiary butyl ether, that contains molybdenum, bismuth and iron as essential elements (see Japanese Unexamined Patent Application Publication Nos. 50-13308, 64-56634, Japanese Examined Patent Application Publication No. 56-23969, Japanese Unexamined Patent Application Publication No. 59-76541, and the like).

(3) A catalyst for producing acrylic acid by oxidizing acrolein, that contains molybdenum and vanadium as essential elements (see Japanese Examined Patent Application Publication No. 49-11371, Japanese Unexamined Patent Application Publication Nos. 52-85091, 6-279030, 8-299797, 2006-297232, and the like).

(4) A catalyst for producing methacrylic acid by oxidizing methacrolein, that contains molybdenum and phosphorus as essential elements (see Japanese Examined Patent Application Publication Nos. 60-33539, 3-26101, Japanese Unexamined Patent Application Publication No. 59-12758, and the like).

(5) A catalyst for producing phthalic anhydride by oxidizing ortho-xylene and/or naphthalene, that contains vanadium and titanium as essential elements (see Japanese Examined Patent Application Publication Nos. 7-29056, 58-15176, and the like).

(6) A catalyst for producing maleic anhydride by oxidizing benzene, that contains molybdenum as an essential element (see Japanese Unexamined Patent Application Publication No. 62-78, and the like).

(7) A catalyst for producing maleic anhydride by oxidizing n-butane, that contains phosphorus and vanadium as essential elements (see Japanese Unexamined Patent Application Publication Nos. 10-167711, 7-51573, 5-115783, 50-35088, and the like).

(8) A catalyst for producing propylene, acrolein, and/or acrylic acid by oxidizing propane, that contains molybdenum as an essential element (see Japanese Unexamined

Patent Application Publication Nos. 9-316023, 10-57813, 10-120617, and the like). (9) A solid particle catalyst which is loaded into a fixed-bed multitubular reactor and which is used for a gas-phase catalytic oxidation reaction, in addition to the above.

It is noted that the catalyst, that is one kind of the solid particles used by being loaded into a fixed-bed multitubular reactor in the present invention, is not limited to the solid particle catalysts described in the above items (1) to (9), that are used for gas-phase catalytic reactions; and includes solid particle catalysts used for ammoxidation reaction, hydrogenation reaction, dehydrogenation reaction, or the like.

As the catalyst, a catalytically active component containing the above-mentioned essential element, depending on the reaction for which the catalyst is used, may be used as it is; or a molded catalyst, which is obtained by molding a catalyst partially mixed with a powdery substance inactive to the reaction, may be used; or a supported catalyst; in which a catalytically active component is supported on a carrier inactive to the reaction, may be used. The method of preparing the catalyst is not particularly restricted and may be appropriately chosen depending on the reaction for which the catalyst is used, and a conventionally well-known method such as those described in the above patent publications may be used.

A shape of the catalyst is not particularly restricted, and a catalyst having a well-known shape such as a cylindrical shape, a ring shape, a spherical shape, or an irregular shape, may be used, for example.

A size of the catalyst is not particularly limited as long as it can be put in the reaction tubes. Concerning the relation between a particle diameter (d) of the catalyst and an inner diameter (D) of the reaction tube in which the catalyst is loaded, the ratio D/d is preferably 2/1 or more, more preferably 2.5/1 or more, and preferably 15/1 or less, more preferably 10/1 or less. The particle diameter (d) of the catalyst means the diameter of a spherical-shaped catalyst, the diameter of a circular cross-section or a multiple circular cross-section of a cylindrical or ring-shaped catalyst, or the longest distance between any two points of a catalyst having any other shape. Further, in the case that the catalyst has a cylindrical or ring shape, the ratio of the diameter (Φ) in the cross-section to the height (h: the length in the direction orthogonal to the cross-section), h/Φ, is preferably in a range of 0.3/1 or more and 3/1 or less. Such a catalyst is easy to be handled and is also preferable in the aspect of production.

In each case of the above-mentioned items (1) to (9), the catalyst which is filled in each layer of the solid particles formed in respective reaction tubes of the fixed-bed multitubular reactor, is not necessarily a single kind, and may be a mixture of a plurality of kinds of catalysts different in a composition, shape or activity, or may be a mixture of a catalyst and an inactive carrier or the like.

An inert substance may be used as the solid particles. The "inert substance" means a substance which is generally inert to a raw material and a target product of a gas-phase catalytic oxidation reaction. Examples of the inert substance include a support for supporting a catalyst used when loading the catalyst into the fixed-bed multitubular reactor; a diluent material for a catalyst; and a substance used as a preheating layer or a cooling layer for a reaction gas. Specific examples thereof include silica, alumina, silica-alumina, and metals (stainless steel, and the like). A shape of the inert substance is not restricted, and examples thereof include, for example, a spherical shape, a ring shape, a Raschig ring shape, and a cylindrical shape. These inert substances may be used either alone or as an appropriate mixture of at least two of them.

A size of the inert substance is not particularly limited as long as it can be put the reaction tubes. An inert substance having a size within the same range as the above-mentioned range of the size of the catalyst may be used.

For an operation method for loading the solid particles into the fixed-bed multitubular reactor, a well-known method may be used. For example, a loading machine disclosed in documents such as Japanese Examined Utility Model Application Publication No. 1-33152, Japanese Examined Patent Application Publication No. 3-9770, Japanese Unexamined Patent Application Publication Nos. 11-333282, 2002-306953, and the like, may be used for the efficient operation.

A number of the layers of the solid particles is also not particularly limited, and about two to five layers will suffice in consideration of the efficient removal of the powdered particles and workability. A total length of the layers of the solid particles loaded into the reaction tube is not particularly limited as long as it is not more than the length of the reaction tube, and can be appropriately chosen within a range not more than the length of the reaction tube.

In the present invention, the gaseous fluid for removing the powdered particles generated upon loading the solid particles into the fixed-bed multitubular reactor is not particularly restricted as long as it does not substantially affect the solid particles to be loaded; and air or nitrogen is preferable from an industrial viewpoint. Particularly, air is preferable in the aspect of safety and economy. Temperature and humidity of the gaseous fluid are also not particularly restricted as long as they do not substantially affect the solid particles to be loaded. Preferably, the gaseous fluid has a temperature in a range of about 1 °C to 60°C and a humidity of 90% or less.

A flow velocity of the gaseous fluid introduced into the reaction tubes can be appropriately determined depending on the shape and loading manners of the solid particles to be loaded such as a catalyst, and is not particularly limited as long as the powdered particles present in the reaction tubes are pushed out of the tubes. For example, when a general reactor used for the gas-phase catalytic oxidation reaction is assumed, the flow velocity (linear velocity) of the gaseous fluid introduced into the reaction tubes is preferably 0.8 m/s or more, more preferably 1.1 m/s or more, and preferably 2.0 m/s or less. When the flow velocity of the gaseous fluid is 0.8 m/s or more, the powdered particles present in the reaction tubes are sufficiently pushed out of the tubes. On the other hand, even when the flow velocity of the gaseous fluid exceeds 2.0 m/s, the effect of pushing out the powdered particles does not improve significantly, and contrarily the solid particles other than the powdered particles may flow and be rubbed with each other, resulting in generating powdered particles. Therefore, the upper limit of the flow velocity of the gaseous fluid is preferably 2.0 m/s. The linear velocity of the gaseous fluid introduced into the reaction tube is calculated by dividing the volume (standard condition) of the gaseous fluid introduced into the reaction tube per unit time by the cross-sectional area of the reaction tube.

A period of introducing the gaseous fluid into the reaction tubes can be appropriately determined depending on the shape and loading manners of the solid particles to be loaded such as a catalyst, and is not particularly limited as long as the powdered particles present in the reaction tubes are pushed out of the tubes. Generally, the period of introducing the gaseous fluid (treatment time) is short when the flow velocity of the gaseous fluid is high, whereas the period is long when the flow velocity of the gaseous fluid is low. When a general reactor used for the gas-phase catalytic oxidation reaction is assumed, the product of the linear velocity of the gaseous fluid and the treatment time is preferably twice or more the layer length of the solid particles filled in the reaction tubes, for example. Specifically, the product of the linear velocity of the gaseous fluid and the treatment time is preferably 10 meters or more, and more preferably 20 meters or more.

In addition, since the powdered particles become harder to be removed as the solid particles are sequentially loaded into the reaction tubes, it is effective to increase the amount of the gaseous fluid introduced into the reaction tubes every time a layer of the solid particles is loaded into the reaction tubes. Specifically, the linear velocity of the gaseous fluid introduced into the reaction tubes and/or the period of introducing the gaseous fluid may be appropriately increased, although it depends on a mechanical strength of the solid particles to be loaded and a length of each layer of the solid particles.

As a matter of course, in the present invention, as long as the gaseous fluid is introduced into the reaction tubes every time a layer of the solid particles is loaded into the reaction tubes, it is also possible to load each layer of the solid particles which has been divided into smaller portions and introduce the gaseous fluid every time the divided portion is loaded. Such an embodiment is also included in the scope of the present invention.

The gaseous fluid may be introduced from either a top or a bottom of the reaction tube, and a position to introduce the gaseous fluid may be appropriately determined depending on the structure of the fixed-bed multitubular reactor and loading manners of the solid particles. Preferably, the gaseous fluid is introduced from the top of the reaction tube. By introducing the gaseous fluid from the top of the reaction tube, it is possible to push the powdered particles effectively out of the reaction tube also due to a sedimentation effect produced by the powdered particles' own weight.

The removal treatment of the powdered particles may be performed for each of the reaction tubes, or may be performed for all the reaction tubes of the fixed-bed multitubular reactor at once.

Further, in the removal treatment of the powdered particles, the powdered particles can be removed more effectively by, for example, vacuuming the reaction tube from an end opposite to the gaseous fluid introducing end by pressure reduction or the like at the same time with the introduction of the gaseous fluid into the reaction tube.

In this case, the reaction tubes may be vacuumed one by one, or all the reaction tubes of the fixed-bed multitubular reactor may be vacuumed at once.

The powdered particles which is ejected by the gaseous fluid from the reaction tubes in which the solid particles has been loaded can be captured by a general method. For example, a bag filter may be installed on an outlet of the reaction tubes in which the gaseous fluid flows, whereby the powdered particles ejected from the reaction tubes may be captured.

The pressure loss is ideally adjusted so that all the reaction tubes of the fixed-bed multitubular reactor may have the same pressure loss. In a fixed-bed multitubular reactor in an industrial scale having thousands or tens of thousands of reaction tubes, however, it is not preferred since substantial effort and time are to be consumed for the adjustment. In the present invention, the pressure losses of the reaction tubes after removal of the powdered particles are adjusted, relative to the average value thereof (average pressure loss), preferably within a range of 85% to 115% ($\pm$15% of the average value), and more preferably within a range of 92% to 108% ($\pm$8% of the average value). By setting the pressure loss within such a range, a target product may be stably obtained at a high yield for a longer period. The average pressure loss is determined exactly by measuring the pressure losses of all the reaction tubes of the fixed-bed multitubular reactor; however, it is also possible to measure the pressure losses of the reaction tubes that correspond to 5% of all the reaction tubes of the fixed-bed multitubular reactor and calculate the average value thereof to serve as the average pressure loss.

In the present invention, the pressure loss obtained after loading the solid particles into the reaction tube and removing the powdered particles, is a pressure value at the top of the reaction tube when gas such as air or nitrogen is introduced into a constant flow rate from the top of the reaction tube in a state where the bottom of the reaction tube is open. The flow rate of the gas such as air or nitrogen used for the measurement is not particularly limited; however, the pressure loss can be appropriately determined in consideration of the flow rate of the gas per reaction tube in conducting an actual reaction.

EXAMPLES

The present invention will hereinafter be described more specifically by reference to Examples and Comparative Examples; however, the present invention is not limited to these Examples. The present invention can be put into practice after appropriate modifications or variations within a range meeting the gist of the present invention, all of which are included in the technical scope of the present invention. Hereinafter, the term "part(s) by mass" may be described simply as "part(s)" for convenience sake.

Evaluation of Performance

Conversion and yield were defined by the following equations:

$$\text{Conversion (mol\%)} = (\text{molar quantity of a reacted raw material}) / (\text{mole quantity of a fed raw material}) \times 100$$

$$\text{Yield (mol\%)} = (\text{molar quantity of a produced target product}) / (\text{mole quantity of a fed raw material}) \times 100$$

Measurement of Pressure Loss

The pressure loss was measured by introducing air of 20°C at a linear velocity of 1.0 m/s from the top of a reaction tube in a state where the bottom of the reaction tube was open.

Reference Example 1

(Preparation of Catalyst) 1,500 parts of distilled water was heated and stirred, and 500 parts of ammonium paramolybdate was dissolved therein to give a solution A. Separately, 398 parts of cobalt nitrate and 199 parts of nickel nitrate were dissolved in 500 parts of distilled water to give a solution B. Further separately, 25 parts of concentrated nitric acid (65%) was added to 100 parts of distilled water to give an acidic solution, and 137 parts of bismuth nitrate and 95 parts of ferric nitrate were dissolved therein to give a solution C. The solutions B and C were added dropwise to the solution A, and then, to the obtained mixture, 3.6 parts of potassium nitrate and 126 parts of tungsten trioxide were added. The thus obtained slurry was heated and stirred to vaporize the solvent. The resultant dry product was dried at 200°C and then pulverized to 150 $\mu$m or less to give a catalyst powder. 30 mass % ammonium nitrate aqueous solution was added to the catalyst powder, and the resultant product was kneaded and extrusion-molded into a ring shape having an outer diameter of 6 mm, an inner diameter of 2 mm, and a length of 6 mm. The ring was calcined at 480°C for 8 hours under air flow to give a catalyst (1). The catalyst (1) had the following metal element composition excluding oxygen.
Catalyst (1) $Mo_{12}Bi_{1.2}W_{2.3}Fe_{1.0}Co_{5.8}Ni_{2.9}K_{0.15}$

Reference Example 2

A catalyst (2) was obtained in the same manner as in Reference Example 1, except that the product was extrusion-molded into a ring shape having an outer diameter of 8 mm, an inner diameter of 2.5 mm, and a length of 8 mm in Reference Example 1. The metal element composition of the catalyst (2) was same as that of the catalyst (1).

Example 1

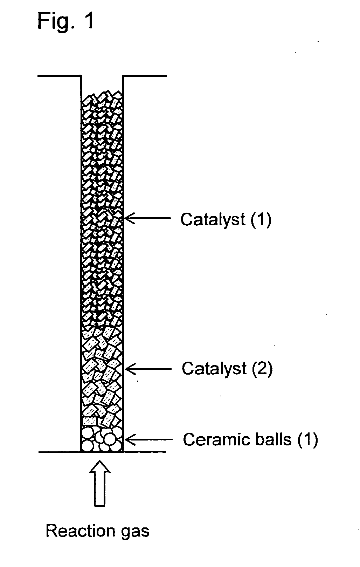

Into a fixed-bed multitubular reactor having 13,000 reaction tubes (reaction tube diameter: 25 mm, length: 3,500 mm) and a shell in which a heat medium is flowed and which covers the reaction tubes, ceramic balls having an average particle diameter of 8 mm, the catalyst (2), and the catalyst (1) were loaded, in this order from the bottom of the reaction tube, by dropping the respective solid particles from the tops of the reaction tubes so that the layer lengths comes to be 150 mm, 850 mm and 2,200 mm, respectively, as shown in Fig. 1. After loading each of the ceramic balls, the catalyst (2) and the catalyst (1), air of 20°C was introduced from the tops of the reaction tubes at a linear velocity of 1.2 m/s and an introducing period of 12 seconds per reaction tube, whereby removal treatments of powdered particles generated during loading were performed. The removed powdered particles were captured by a bag filter installed to the bottoms of the reaction tubes. After the removal of powdered particles was completed, an average pressure loss of all the reaction tubes was measured. The measurement result is shown in Table 1.

Example 2

Solid particles were loaded into the reactor in the same manner as in Example 1, except that the introducing period of air that was introduced into the reaction tubes for removing the powdered particles after loading the catalyst (1) was changed to 16 seconds in Example 1. The result is shown in Table 1.

Example 3

Solid particles were loaded into the reactor in the same manner as in Example 1, except that the introducing period of air which was introduced into the reaction tubes for removing the powdered particles after loading the catalyst (2) was changed to 15 seconds, and the linear velocity of air which was introduced into the reaction tubes for removing the powdered particles after loading the catalyst (1) was changed to 1.5 m/s in Example 2. The result is shown in Table 1.

Example 4

Solid particles were loaded into the reactor in the same manner as in Example 3, except that the loading of the catalysts and the removal of the powdered particles were performed while vacuuming the reaction tubes from the bottom thereof by using an exhaust suction apparatus in Example 3. The result is shown in Table 1.

Comparative Example 1

Solid particles were loaded into the reactor exactly in the same manner as in Example 1, except that the removal treatment of the powdered particles by introducing air was not conducted in Example 1, and the average pressure loss at that time was measured. The result is shown in Table 1. Since the removal treatment of the powdered particles by introducing air was not conducted in Comparative Example 1, the average pressure loss in Comparative Example 1 was high as compared with those in Examples 1 to 4.

Reference Example 3

3,000 parts of distilled water was heated and stirred, and 500 parts of ammonium molybdate, 83 parts of ammonium paratungstate, and 166 parts of ammonium metavanadate were dissolved therein. Separately, 200 parts of cupper nitrate was dissolved in 185 parts of distilled water. The thus two obtained solutions were mixed, and 10 parts of antimony trioxide was added thereto to give a suspension. The obtained suspension was evaporated to dryness, and further, the resultant dry product was calcined at 350°C for about 4 hours under an air atmosphere, and then, pulverized to 150 $\mu$m or less to give a catalyst powder. 1300 parts of a silica-alumina spherical carrier having an average particle diameter of 4.5 mm was put in a tumbling granulator, and then, 5 mass % ammonium nitrate aqueous solution as a binder and the catalyst powder were gradually put therein, whereby the catalyst powder was supported on the surface of the carrier. And then, the product was calcined at 400°C for 6 hours under an air atmosphere to give a catalyst (3). The catalyst (3) had the following metal element composition excluding oxygen and the carrier.
Catalyst (3) $Mo_{12}V_{6.0}W_{1.3}Cu_{3.5}Sb_{0.3}$

Reference Example 4

A catalyst (4) was prepared in the same manner as in Reference Example 3, except that the amount of ammonium metavanadate was changed to 127 parts, 34 parts of cobalt nitrate was added together with copper nitrate, and the carrier was changed to a silica-alumina spherical carrier having an average particle diameter of 7.0 mm in Reference Example 3. The catalyst (4) had the following metal element composition excluding oxygen and the carrier.
Catalyst (4) $Mo_{12}V_{4.6}W_{1.3}Cu_{3.5}Co_{0.5}Sb_{0.3}$

Example 5

Solid particles were loaded into the reactor and powdered particles were removed in the same manner as in Example 1, except that ceramic balls having an average particle diameter of 8 mm, the catalyst (4), and the catalyst (3) were loaded into the same fixed-bed multitubular reactor as Example 1, in this order from the bottom of the reaction tube, so that the layer lengths comes to be 150 mm, 900 mm and 1,800 mm, respectively. After the removal of powdered particles, the average pressure loss of all the reaction tubes was measured. The measurement result is shown in Table 1.

Example 6

Solid particles were loaded into the reactor in the same manner as in Example 5, except that the linear velocity of air which was introduced into the reaction tubes for removing the powdered particles after loading the catalyst (4) was changed to 1.5 m/s, and the linear velocity and the introducing period of air which was introduced into the reaction tubes for removing the powdered particles after loading the catalyst (3) was changed to 1.5 m/s and 18 seconds, respectively, in Example 5. The result is shown in Table 1.

Example 7

Solid particles were loaded into the reactor in the same manner as in Example 6, except that the loading of the catalysts and the removal of the powdered particles were performed while vacuuming the reaction tubes from the bottom thereof by using an exhaust suction apparatus in Example 6. The result is shown in Table 1.

Comparative Example 2

Solid particles were loaded into the reactor in the same manner as in Example 5, except that the removal treatment of the powdered particles was not conducted in Example 5, and the average pressure loss at that time was measured. The result is shown in Table 1. Since the removal treatment of the powdered particles by introducing air was not conducted in Comparative Example 2, the average pressure loss in Comparative Example 2 was high as compared with those in Examples 5 to 7.

Table 1

|  | Average Pressure Loss (Pa) |
|---|---|
| Example 1 | 2910 |
| Example 2 | 2860 |
| Example 3 | 2810 |
| Example 4 | 2770 |
| Comparative Example 1 | 3060 |
| Example 5 | 2530 |
| Example 6 | 2470 |
| Example 7 | 2420 |
| Comparative Example 2 | 2700 |

Example 8

A top outlet of the reactor in Example 4 (referred to as a "first reactor") and a bottom inlet of the reactor in Example 7 (referred to as a "second reactor") were connected by a steel pipe, which had an inner diameter of 500 mm and a length of 5,000 mm, and which was enabled to be heated by steam from outside. A mixed gas, composed of 8.2 volume % of propylene, 15 volume % of oxygen, 13.8 volume % of steam and 63 volume % of an inert gas such as nitrogen, was introduced from the bottom of the first reactor at a space velocity (STP) of 1550 $h^{-1}$ relative to the catalysts filled in the first reactor, whereby an oxidation reaction of propylene was conducted successively for 8000 hours. The result is shown in Table 2.

Example 9

The oxidation reaction of propylene was conducted successively for 8000 hours exactly in the same manner as in Example 8, except that the action to bring the pressure losses of the reaction tubes into the range of 85% to 115% was taken by partially extracting the catalyst from the uppermost layer in the reaction tubes whose pressure losses were higher than 115% of the average pressure loss of all the reaction tubes and adding the catalyst to the uppermost layer in the reaction tubes whose pressure losses were lower than 85% of the average pressure loss in Example 8. The result is shown in Table 2. In Example 9, by measuring and adjusting the pressure loss of the each reaction tube after introducing the gaseous fluid into the reaction tubes, the average pressure loss after 8000 hours reduced, and the conversion of propylene and the yield of acrylic acid improved, as compared with Example 8.

Comparative Example 3

The oxidation reaction of propylene was conducted successively for 8000 hours in the same manner as in Example 8, except that the reactor described in Comparative Example 1 was used as the first reactor and the reactor described in Comparative Example 2 was used as the second reactor. The result is shown in Table 2. In Comparative Example 3, the average pressure loss after 8000 hours increased significantly, and the conversion of propylene and the yield of acrylic acid reduced, as compared with Examples 8 and 9.

Example 10

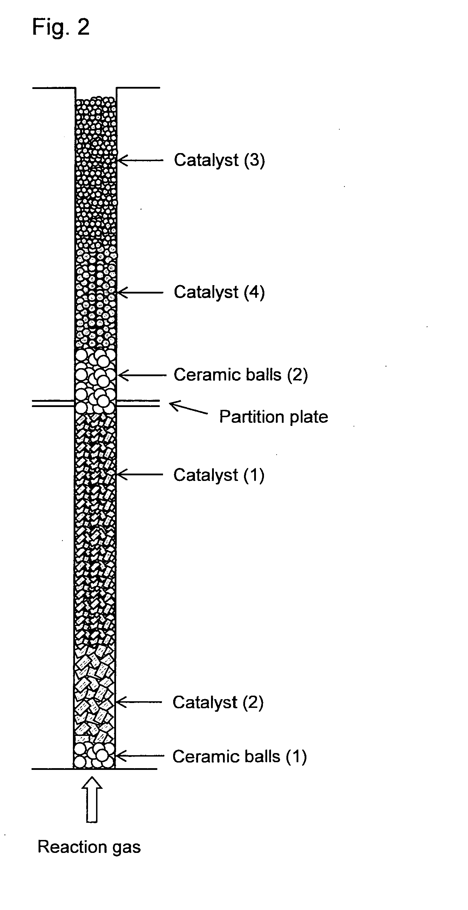

Into a fixed-bed multitubular reactor having about 9,500 reaction tubes (reaction tube diameter: 25 mm, length: 6,500 mm) and a shell in which a heat medium is flowed and which covers the reaction tubes, ceramic balls (1) having an average particle diameter of 8 mm, the catalyst (2), the catalyst (1), ceramic balls (2) having an average particle diameter of 8 mm, the catalyst (4), and the catalyst (3) were loaded by sequentially dropping them in this order from the tops of the reaction tubes so that the layer lengths comes to be 150 mm for the ceramic balls (1), 850 mm for the catalyst (2), 2,200 mm for the catalyst (1), 450 mm for the ceramic balls (2), 900 mm for the catalyst (4), and 1,800 mm for the catalyst (3), as shown in Fig. 2. After loading each kind of the particles, air of 20°C was introduced from the tops of the reaction tubes at the following linear velocity and for the following introducing period per reaction tube, whereby powdered particles generated by loading the solid particles were removed: the ceramic balls (1): 1.2 m/s for 12 seconds; the catalyst (2): 1.2 m/s for 15 seconds; the catalyst (1): 1.5 m/s for 16 seconds; the ceramic balls (2): 1.5 m/s for 18 seconds; the catalyst (4): 1.8 m/s for 18 seconds; and the catalyst (3): 1.8 m/s for 22 seconds. The powdered particles discharged from the reaction tubes were captured by a bag filter installed to the bottom of the reactor. After the catalyst (3) was loaded and the removal of the powdered particles was completed, the average pressure loss of all the reaction tubes was measured. A partition plate having a thickness of 50 mm that divides the shell into upper and lower shell spaces was also installed at a position 3,250 mm from the bottom of the shell, and the heat medium was circulated from bottom to top in the both upper and lower shell spaces. A reaction zone filled with the oxide catalysts (1) and (2) is referred as a first reaction zone, and a reaction zone filled with the oxide catalysts (3) and (4) is referred as a second reaction zone. A mixed gas, composed of 8.3 volume % of propylene, 15.2 volume % of oxygen, 14.5 volume % of steam and 62 volume % of an inert gas such as nitrogen, was introduced from the bottom of the fixed-bed multitubular reactor in which the solid particles are loaded in the manner as described above at a space velocity (STP) of 1600 h$^{-1}$ relative to the catalysts filled in the first reaction zone, whereby the oxidation reaction of propylene was conducted successively for 8000 hours. The result is shown in Table 3.

Example 11

The oxidation reaction of propylene was conducted successively for 8000 hours under the same condition in exactly the same fixed-bed multitubular reactor as in Example 10, except that the action to bring the pressure losses of the reaction tubes into the range of 92% to 108% was taken by partially extracting the catalyst (3) from the reaction tubes whose pressure losses were higher than 108% of the average pressure loss of all the reaction tubes and adding the catalyst (3) to the reaction tubes whose pressure losses were lower than 92% of the average pressure loss in Example 10. The result is shown in Table 3. In Example 11, by measuring and adjusting the pressure loss of the each reaction tube after introducing the gaseous fluid into the reaction tubes, the average pressure loss reduced, and the conversion of propylene and the yield of acrylic acid improved, as compared with Example 10.

Comparative Example 4

The oxidation reaction of propylene was conducted successively for 8000 hours exactly in the same manner as in Example 10, except that the removal treatment of the powdered particles generated by loading the solid particles was not performed. The result at the beginning of the reaction and after 8000 hours are shown in Table 3. In Comparative Example 4, the average pressure loss after 8000 hours increased significantly, and the conversion of propylene and the yield of acrylic acid reduced, as compared with Examples 10 and 11.

Comparative Example 5

The oxidation reaction of propylene was conducted successively for 8000 hours under the same condition in exactly the same fixed-bed multitubular reactor as in Example 10, except that the treatment of the powdered particles generated by loading the solid particles was not performed, and then, the pressure losses of the reaction tubes were adjusted to be within the range of 85% to 115% relative to the average pressure loss of all the reaction tubes in Example 10. The result is shown in Fig. 3. In Comparative Example 5, the average pressure loss after 8000 hours increased significantly, and the conversion of propylene and the yield of acrylic acid reduced, as compared with Examples 10 and 11.

Table 2

| | Passage of Time | Average Pressure Loss (Pa) | | | | Reaction Temperature (°C) | | Conversion of Propylene (mol%) | Yield of Acrylic Acid (mol%) |
| | | First Reactor | Distribution of Pressure Loss | Second Reactor | Distribution of Pressure Loss | First Reactor | Second Reactor | | |
|---|---|---|---|---|---|---|---|---|---|
| Example 8 | beginning of reaction | 2770 | ±16% | 2420 | ±16% | 305 | 263 | 97.1 | 93.3 |
| | after 8000 hours | 2900 | ±17% | 2540 | ±18% | 313 | 273 | 96.9 | 92.7 |
| Example 9 | beginning of reaction | 2790 | ±7% | 2430 | ±6% | 302 | 261 | 97.5 | 93.9 |
| | after 8000 hours | 2880 | ±8% | 2490 | ±8% | 308 | 269 | 97.3 | 93.6 |
| Comparative Example 3 | beginning of reaction | 3060 | ±17% | 2700 | ±18% | 308 | 267 | 96.7 | 92.3 |
| | after 8000 hours | 3300 | ±22% | 2870 | ±24% | 324 | 283 | 96.3 | 91.2 |

Table 3

| | Passage of Time | Average Pressure Loss (Pa) | Distribution of Pressure Loss | Reaction Temperature (°C) | | Conversion of Propylene (mol%) | Yield of Acrylic Acid (mol%) |
|---|---|---|---|---|---|---|---|
| | | | | First Reaction Zone | Second Reaction Zone | | |
| Example 10 | beginning of reaction | 5640 | ±13% | 303 | 262 | 97.2 | 93.5 |
| | after 8000 hours | 5910 | ±15% | 310 | 272 | 97.1 | 93.1 |
| Example 11 | beginning of reaction | 5630 | ±5% | 301 | 260 | 97.7 | 94.2 |
| | after 8000 hours | 5810 | ±6% | 306 | 268 | 97.6 | 93.9 |
| Comparative Example 4 | beginning of reaction | 6150 | ±18% | 309 | 266 | 96.9 | 92.6 |
| | after 8000 hours | 6560 | ±25% | 325 | 281 | 96.8 | 91.3 |
| Comparative Example 5 | beginning of reaction | 6090 | ±8% | 305 | 265 | 97.0 | 92.9 |
| | after 8000 hours | 6520 | ±17% | 321 | 277 | 96.7 | 91.6 |

**Claims**

1. A method of loading solid particles into reaction tubes of a fixed-bed multitubular reactor, comprising the step of:

   loading two or more kinds of solid particles into each of the reaction tubes so that the respective kinds of the solid particles are stacked in layers in a direction of an axis of the tube,
   wherein a gaseous fluid is introduced into the reaction tubes at least once every time the each kind of the solid particles is loaded in the layer.

2. The method of loading solid particles according to claim 1, wherein at least the one kind of the solid particles is a catalyst.

3. The method of loading solid particles according to claim 1 or 2, wherein
   the gaseous fluid is introduced into the reaction tubes at a linear velocity of 0.8 m/s or more, and
   a product of the linear velocity and a period introducing the gaseous fluid into the reaction tubes is 10 meters or more.

4. The method of loading solid particles according to any one of claims 1 to 3, wherein an amount of the gaseous fluid introduced into the reaction tubes increases depending on a number of the layers of the solid particles loaded into the reaction tube.

5. The method of loading solid particles according to any one of claims 1 to 4, wherein the gaseous fluid is air.

6. The method of loading solid particles according to any one of claims 1 to 5, wherein a pressure loss of the each reaction tube is measured and adjusted after introducing the gaseous fluid.

## Fig. 1

Catalyst (1)

Catalyst (2)

Ceramic balls (1)

Reaction gas

Fig. 2

Catalyst (3)

Catalyst (4)

Ceramic balls (2)

Partition plate

Catalyst (1)

Catalyst (2)

Ceramic balls (1)

Reaction gas

## INTERNATIONAL SEARCH REPORT

| | International application No. |
|---|---|
| | PCT/JP2009/061096 |

A.  CLASSIFICATION OF SUBJECT MATTER
B01J8/02(2006.01)i, C07C51/25(2006.01)n

According to International Patent Classification (IPC) or to both national classification and IPC

B.  FIELDS SEARCHED

Minimum documentation searched (classification system followed by classification symbols)
B01J8/02, C07C51/25

Documentation searched other than minimum documentation to the extent that such documents are included in the fields searched
Jitsuyo Shinan Koho        1922–1996   Jitsuyo Shinan Toroku Koho   1996–2009
Kokai Jitsuyo Shinan Koho   1971–2009   Toroku Jitsuyo Shinan Koho   1994–2009

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y | JP 2003-252807 A  (Mitsubishi Chemical Corp.), 10 September, 2003 (10.09.03), Par. Nos. [0001], [0048] to [0049], [0076] to [0078] & US 2004/0250868 A1    & EP 1466883 A1 & WO 2003/057653 A1    & CN 1607030 A & CN 1607031 A        & CN 1607032 A & CN 1617843 A | 1-6 |
| Y | JP 2004-195278 A  (Mitsubishi Chemical Corp.), 15 July, 2004 (15.07.04), Par. Nos. [0001], [0026] (Family: none) | 1-6 |

[X] Further documents are listed in the continuation of Box C.     [ ] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search 24 August, 2009 (24.08.09) | Date of mailing of the international search report 15 September, 2009 (15.09.09) |
|---|---|
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2009/061096

C (Continuation).    DOCUMENTS CONSIDERED TO BE RELEVANT

| Category* | Citation of document, with indication, where appropriate, of the relevant passages | Relevant to claim No. |
|---|---|---|
| P,X | JP 2008-246284 A (Sumitomo Chemical Co., Ltd.), 16 October, 2008 (16.10.08), Claims 1 to 4; Par. Nos. [0011] to [0018] (Family: none) | 1-6 |
| A | JP 10-277381 A (Mitsubishi Rayon Co., Ltd.), 20 October, 1998 (20.10.98), Full text (Family: none) | 1-6 |
| A | JP 2008-36527 A (Mitsubishi Rayon Co., Ltd.), 21 February, 2008 (21.02.08), Full text (Family: none) | 1-6 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003340266 A
- JP 6007669 A
- JP 62030545 A
- JP 2005224661 A
- JP 63116743 A
- JP 62004444 A
- JP 5329368 A
- JP 10510212 A
- JP 5084440 A
- JP 50013308 A
- JP 6456634 B
- JP 56023969 A
- JP 59076541 A
- JP 49011371 A
- JP 52085091 A
- JP 6279030 A
- JP 8299797 A
- JP 2006297232 A
- JP 60033539 A
- JP 3026101 A
- JP 59012758 A
- JP 7029056 A
- JP 58015176 A
- JP 62000078 A
- JP 10167711 A
- JP 7051573 A
- JP 5115783 A
- JP 50035088 A
- JP 9316023 A
- JP 10057813 A
- JP 10120617 A
- JP 1033152 A
- JP 3009770 A
- JP 11333282 A
- JP 2002306953 A